# EUROPEAN PATENT APPLICATION

(11) **EP 4 410 960 A1**
(43) Date of publication of application: **07.08.2024**
(21) Application number: 22875771.2
(22) Date of filing: 08.09.2022
(51) Int. Cl.: C12N 5/071, C12N 15/86, C12N 15/864

(54) **AGENT FOR INDUCING VIRAL VECTOR PRODUCTION**

(30) Priority: 01.10.2021 JP 2021162665
(71) Applicant: Kaneka Corporation, Osaka-shi, Osaka 530-8288 (JP)
(72) Inventor: SASAMOTO, Koki, Takasago-shi, Hyogo 676-8688 (JP); SASAKI, Takahide, Takasago-shi, Hyogo 676-8688 (JP); MAEDA, Hirofumi, Takasago-shi, Hyogo 676-8688 (JP); KITANO, Mitsuaki, Takasago-shi, Hyogo 676-8688 (JP)
(74) Representative: Hoffmann Eitle
(86) International application number: PCT/JP2022/033743
(87) International publication number: WO 2023/053899

(57) **Abstract**

Provided are an agent for inducing viral vector production, comprising cell growth inhibitor a, wherein said cell growth inhibitor a comprises a compound which inhibits progression of cell cycle in G2 phase or M phase; a method of producing a viral vector and a method of inducing viral vector production using the same; and a method of producing a viral vector and a method of inducing viral vector production comprising a step of introducing a nucleic acid into a cell and a step of adding cell growth inhibitor A to said cell wherein said cell growth inhibitor A is added between 6 hours before and 6 hours after the time of introducing said nucleic acid.

## Description

### Technical Field

The present invention relates to an agent for inducing viral vector production.

### Background Art

Viral vectors are widely used in the gene therapy. Viral vectors are those in which a gene of a wild-type virus is replaced with a foreign gene such as a fluorescent protein expression gene or a therapeutic protein expression gene.

With respect to methods for viral vector production, such as a method of introducing a nucleic acid for expression of a viral vector into cells and producing viral vector production cells, and purifying a viral vector from the cell supernatant or cell suspension thereof, it is a problem that viral vectors are not sufficiently produced in viral vector production cells.

As a solution for solving this problem, a method of using a histone deacetylase (HDAC) inhibitor as an agent for inducing production is known (Patent Literature 1). However, this method is not very useful, since further increases are not observed when the amount of viral vector production (titer) is high in the viral vector production cells.

Additionally, nocodazole-DMSO is disclosed as an AAV production enhancer (Patent Literature 2). However, concentration of nocodazole-DMSO, timing of addition of nocodazole-DMSO, type of solubilizer, and type of nucleic acid to be introduced into a cell are not examined. Furthermore, it is known that nocodazole reduces the productivity of virus-like particles (Non Patent Literature 1).

Accordingly, an agent for inducing viral vector production comprising a compound which inhibits progression of cell cycle in G2 phase or M phase for improving viral vector productivity, as well as a method of producing a viral vector and a method of inducing viral vector production which use the same agent, and a method of producing a viral vector and a method of inducing viral vector production for improving viral vector productivity are not known at all, and there are strong needs for them.

### Citation List

### Patent Literature

Patent Literature 1: JP2020-524498A
Patent Literature 2: WO2020/172624

### Non Patent Literature

Non Patent Literature 1: Applied. Microbiology and Biotechnology 2015 99:9935-9949

### Summary of Invention

### Technical Problem

The present invention is aimed at solving the conventional problems described above and achieving the following object. Specifically, an object of the present invention is to provide an agent for inducing viral vector production comprising a compound which inhibits progression of cell cycle in G2 phase or M phase for improving viral vector productivity; a method of producing a viral vector and a method of inducing viral vector production which use the same agent; and a method of producing a viral vector and a method of inducing viral vector production for improving viral vector productivity.

### Solution to Problem

The present inventors made intensive studies in order to achieve the object described above, and as a result, found that an agent for inducing viral vector production comprising cell growth inhibitor a, wherein said growth inhibitor a comprises a compound which inhibits progression of cell cycle in G2 phase or M phase, can provide an agent for inducing viral vector production comprising a compound which inhibits progression of cell cycle in G2 phase or M phase for improving viral vector productivity, as well as a method of producing a viral vector and a method of inducing viral vector production which use the same agent; and found that a method of producing a viral vector and a method of inducing viral vector production which comprise a step of introducing a nucleic acid into a cell and a step of adding cell growth inhibitor A to said cell, wherein said cell growth inhibitor A is added between 6 hours before and 6 hours after the time of introducing said nucleic acid, can provide a method of producing a viral vector and a method of inducing viral vector production for improving viral vector productivity.

The present invention is based on the findings by the present inventors as described above, and means for solving the above problems are as follows.
<1> An agent for inducing viral vector production, comprising cell growth inhibitor a, wherein said cell growth inhibitor a comprises a compound which inhibits progression of cell cycle in G2 phase or M phase.
<2> A method of producing a viral vector, wherein said method uses the agent for inducing viral vector production of <1>.
<3> A method of producing a viral vector, comprising a step of introducing a nucleic acid into a cell, and a step of adding cell growth inhibitor A to said cell, wherein said cell growth inhibitor A is added between 6 hours before and 6 hours after the time of introducing said nucleic acid.
<4> A method of inducing viral vector production, wherein said method uses the agent for inducing viral vector production of <1>.
<5> A method of inducing viral vector production, comprising a step of introducing a nucleic acid into a cell, and a step of adding cell growth inhibitor A to said cell, wherein said cell growth inhibitor A is added between 6 hours before and 6 hours after the time of introducing said nucleic acid.

### Advantageous Effects of Invention

The present invention can solve the conventional problems described above and achieve the object described above, and can provide an agent for inducing viral vector production comprising a compound which inhibits progression of cell cycle in G2 phase or M phase for improving viral vector productivity, and a method of producing a viral vector and a method of inducing viral vector production which use the same agent.

### Brief Description of Drawing

[Figure 1] Figure 1 shows the influence of the final concentration of nocodazole on the titer of adeno-associated virus in Example 1.
[Figure 2] Figure 2 shows the influence of the timing of addition of cell growth inhibitor A on the titer of adeno-associated virus in Example 2.
[Figure 3] Figure 3 shows the influence of addition of various cell growth inhibitors A on the titer of adeno-associated virus in Example 3 (Part 1).
[Figure 4] Figure 4 shows the influence of addition of various cell growth inhibitors A on the titer of adeno-associated virus in Example 3 (Part 2).
[Figure 5] Figure 5 shows the influence of addition of various cell growth inhibitors A on the titer of adeno-associated virus in Example 3 (Part 3).
[Figure 6] Figure 6 shows the influence of addition of various cell growth inhibitors A on the titer of adeno-associated virus in Example 3 (Part 4).
[Figure 7] Figure 7 shows the influence of addition of various cell growth inhibitors A on the titer of adeno-associated virus in Example 3 (Part 5).
[Figure 8] Figure 8 shows the influence of addition of sodium valproate on the titer of adeno-associated virus in Comparative Example 1.
[Figure 9] Figure 9 shows the influence of the presence of cell growth inhibitor A on the titer of adeno-associated virus produced using a bioreactor in Example 4.
[Figure 10] Figure 10 shows the influence of using cell growth inhibitor A and linear covalently closed DNAs on the titer of adeno-associated virus in Example 5 (Part 1).
[Figure 11] Figure 11 shows the influence of using cell growth inhibitor A and linear covalently closed DNAs on the titer of adeno-associated virus in Example 5 (Part 2).
[Figure 12] Figure 12 shows the vector map of pAAV-Venus_telRL_ara_TelN in Production Example 2-2.
[Figure 13] Figure 13 shows the vector map of pRC2-mi342_telRL_ara_TelN in Production Example 2-3.
[Figure 14] Figure 14 shows the vector map of pHelper_telRL_ara_TelN in Production Example 2-4.
[Figure 15] Figure 15 shows the evaluation results of DNA by electrophoresis in Production Example 2-6.
[Figure 16] Figure 16 shows the evaluation results of DNA by electrophoresis in Production Example 2-7.

### Description of Embodiments

### (Agent for inducing viral vector production)

The agent for inducing viral vector production comprises cell growth inhibitor a, and can further comprise an additional component.

### <Viral vector>

The viral vector is not particularly limited and can be selected as appropriate depending on the purpose, and examples thereof include an adeno-associated virus (AAV) vector, adenovirus vector, retrovirus vector, lentivirus vector, herpesvirus vector, poliovirus vector, papillomavirus vector, vaccinia virus vector, poxvirus vector, simian virus vector, and Sendai virus vector.

Among them, an adeno-associated virus (AAV) vector is preferred because of low pathogenicity.

Examples of the serotype of the AAV include AAV1 (Type 1 AAV), AAV2 (Type 2 AAV), AAV3 (Type 3 AAV), AAV4 (Type 4 AAV), AAV5 (Type 5 AAV), AAV6 (Type 6 Type AAV), AAV7 (Type 7 AAV), AAV8 (Type 8 AAV), AAV9 (Type 9 AAV), AAV10 (Type 10 AAV), AAV11 (Type 11 AAV), AAV12 (Type 12 AAV), AAV13 (Type 13 AAV), AAV14 (Type 14 AAV), and any modified product thereof, but not particularly limited thereto, and can be selected as appropriate depending on the purpose.

The modified product described above is not particularly limited and can be appropriately selected depending on the purpose, and examples thereof include AAV obtained by modification (of wild-type AAV) by genetic recombination, *e.g.,* for improvement of tissue specificity of target cells (tropism of infected cells).

### <Cell growth inhibitor a>

The cell growth inhibitor a comprises a compound which inhibits progression of cell cycle in G2 phase or M phase. The compound may be used as a single kind alone or as a mixture of two or more kinds.

The compound which inhibits progression of cell cycle in G2 phase or M phase is not particularly limited and can be appropriately selected depending on the purpose, and examples thereof include, *e.g.,* a compound which inhibits microtubule polymerization and a compound which stabilizes microtubules.

Among them, a compound which inhibits microtubule polymerization is preferred from the viewpoint of the effect of inducing viral vector production.

The compound which inhibits microtubule polymerization is not particularly limited and can be selected as appropriate depending on the purpose, and examples thereof include benzimidazole derivative, vinca alkaloid compound, and colchicine derivative.

The benzimidazole derivative is not particularly limited as long as it is various compounds having a benzimidazole ring, and can be selected as appropriate depending on the purpose, and examples thereof include nocodazole, albendazole, mebendazole, thiabendazole, fenbendazole, triclabendazole, flubendazole, oxibendazole, parbendazole, oxfendazole, ricobendazole, or a salt thereof. Among them, nocodazole, albendazole, mebendazole, thiabendazole, fenbendazole, triclabendazole, flubendazole, oxibendazole, parbendazole, or a salt thereof is preferred.

The vinca alkaloid compound is not particularly limited and can be selected as appropriate depending on the purpose, and examples thereof include vinblastine, vincristine, vindesine, vinorelbine, vinflunine, or a derivative of said compound or a salt thereof. Among them, vinblastine or a salt thereof is preferred.

The colchicine derivative is not particularly limited and can be selected as appropriate depending on the purpose, and examples thereof include colchicine, colcemid (demecolcine), allocolchicine, thiocolchicine, or a salt thereof. Among them, colcemid or a salt thereof is preferred.

Among them, nocodazole, albendazole, mebendazole, oxibendazole vinblastine, colcemid or a salt thereof is preferred from the viewpoint of the effect of inducing viral vector production.

As the compound which inhibits microtubule polymerization, a salt form can be suitably used, and examples of the salt form include hydrochloride and sulfate. Specific examples of said salt form include vinblastine sulfate and vincristine sulfate.

As the compound which stabilizes microtubules is not particularly limited and can be selected as appropriate depending on the purpose, and examples thereof include a taxane derivative.

The taxane derivative is not particularly limited as long as it is various compounds having a taxane ring, and can be selected as appropriate depending on the purpose, and examples thereof include paclitaxel and docetaxel.

### <Additional components

The additional component described above is not particularly limited and can be selected as appropriate depending on the purpose, and examples thereof include a solubilizer, a surfactant, a colorant, a stabilizer, and a pH adjuster. These components may be used as a single kind alone or as a mixture of two or more kinds thereof.

Examples of the solubilizer include *e.g.,* water, an acidic aqueous solution, an alcohol, dimethyl sulfoxide (DMSO), dimethylformamide (DMF), dimethylacetamide (DMA), hexamethylphosphoric triamide (HMPA), acetonitrile, acetone, dioxane, and tetrahydrofuran (THF).

The acidic aqueous solution is not particularly limited and can be selected as appropriate depending on the purpose, and examples thereof include an aqueous solution of an acidic compound such as hydrochloric acid, sulfuric acid, nitric acid, phosphoric acid, acetic acid, monofluoroacetic acid, difluoroacetic acid, trifluoroacetic acid, formic acid, oxalic acid, malonic acid, or succinic acid. The acidic compound may be used as a single kind alone or as a mixture of two or more kinds thereof.

The concentration of the acidic aqueous solution is more than 0 and is less than the maximum solubility which is unique to the acidic compound. Specifically, the concentration of said acidic compound is more than 0 w/w% and is equal to or less than the saturated concentration (w/w%) of the acidic compound at 20°C and ordinary pressure.

The alcohol is not particularly limited as long as it is various compounds having a hydroxy group, and can be selected as appropriate depending on the purpose, and examples thereof include *e.g.,* methanol, ethanol, propanol, isopropanol, n-butyl alcohol, isobutyl alcohol, sec-butyl alcohol, and tert-butyl alcohol.

Among them, water, hydrochloric acid, formic acid aqueous solution, ethanol or DMSO is preferred from the viewpoint of the effect of inducing viral vector production and solubility of the compound.

### (Method of producing viral vector 1)

A first aspect of the method of producing a viral vector is a method using an agent for inducing viral vector production comprising cell growth inhibitor a.

The method using an agent for inducing viral vector production is not particularly limited and can be selected as appropriate depending on the purpose, and examples thereof include a method of adding the agent for inducing viral vector production to a viral vector production cell.

The viral vector production cell is not particularly limited and can be selected as appropriate depending on the purpose, and examples thereof include those which are obtained by introducing (transfecting) a nucleic acid for expression of a viral vector into a cell.

The cell described above is not particularly limited and can be selected as appropriate depending on the purpose, and examples thereof include *e.g.,* a mammalian cell and an insect cell.

Among them, a mammalian cell is preferred.

The mammalian cell is not particularly limited and can be selected as appropriate depending on the purpose, and examples thereof include *e.g.,* a human-derived cell, a mouse-derived cell, a hamster-derived cell, a rat-derived cell, a dog-derived cell, a monkey-derived cell, and a kangaroo-derived cell.

Among them, HEK293 cell, CHO cell, Hela cell, Vero cell, BHK cell, COS cell, MDCK cell, 3T3 cell, HepG2 cell, A549 cell, C2C12 cell, C6 cell, HT-1080 cell, Huh-7 cell, H9C2 cell, HCT116 cell, HT-29 cell, K562 cell, LNCaP cell, Jurkat cell, L6 cell, USO2 cell, or PC12 cell is preferred, and HEK293 cell is more preferred, from the viewpoint of the yield of viral vector and safety of the viral vector produced.

The cell described above includes a cell line derived from a parent cell. For example, a HEK293 cell-derived HEK293T cell, a FreeStyle^{™} 293F cell, or Viral Production Cells 2.0 can be used as the HEK293 cell.

The insect cell is not particularly limited and can be selected as appropriate depending on the purpose, and examples thereof include a moth-derived cell and a fly-derived cell.

Among them, Sf-9 cell, Sf-21 cell, High five cell, Tni cell, or S2 cell is preferred.

The cell includes a cell line derived from a parent cell.

The method of culturing a cell is not particularly limited and can be selected as appropriate depending on the purpose, and any of adhesion culture and suspension culture can be used.

Among them, suspension culture is preferably used from the viewpoint of the yield per volume of a culture medium and ease of operation.

The "nucleic acid" can also be called "polynucleotide", and examples thereof include deoxyribonucleic acid (DNA) and ribonucleic acid (RNA), and DNA is preferred. The DNA may be a single strand, double strand, or triple strand, and double-stranded DNA is preferred. The structure of the double-stranded DNA is not particularly limited and can be selected as appropriate depending on the purpose, and examples include *e.g.,* a circular plasmid DNA, a linear plasmid DNA, an open circular plasmid DNA, a circular DNA, a linear DNA, and a linear covalently closed DNA.

Among them, a circular plasmid DNA, a circular DNA, or a linear covalently closed DNA is preferred, and a linear covalently closed DNA is more preferred from the viewpoint of gene transduction efficiency.

The "linear covalently closed DNA" is a linear double-stranded DNA having an end the structure of which is closed in a hairpin configuration, and can also be abbreviated as "LCC DNA".

The method of introducing a nucleic acid as described above is not particularly limited and can be selected as appropriate depending on the purpose, and examples thereof include a method using a cationic polymer such as polyethylenimine (PEI), a method using liposome, a method using calcium phosphate, a method using a branched organic compound such as a dendrimer, a method using diethylaminoethyl (DEAE)-dextran, an electroporation method, a particle gun method, a microinjection method, a magnetofection method, and a method using a viral vector.

Among them, a method using a cationic polymer is preferred considering a high introduction efficiency and considering that the reagent is inexpensive, and also considering that no special apparatus is necessary.

The timing of addition of the agent for inducing viral vector production is not particularly limited, and can be selected as appropriate depending on the purpose, and is preferably between 6 hours before and 6 hours after the time of introducing the nucleic acid, more preferably between 4 hours before and 4 hours after the time of introducing the nucleic acid, further preferably between 5 minutes before and 4 hours after the time of introducing the nucleic acid, still further preferably between 5 minutes before and 2 hours after the time of introducing the nucleic acid, particularly preferably between the same time as and 2 hours after the time of introducing the nucleic acid, from the viewpoint of convenience, and improvement of the effect of inducing viral vector production.

The number of addition of the agent for inducing viral vector production is not particularly limited, and can be selected as appropriate depending on the purpose. For example, the total amount can also be added at one time, or can also be divided into a plurality of doses for the addition.

The method of introducing the nucleic acid is not particularly restricted, and a complex of an introduction reagent (transfection reagent) and the nucleic acid can be formed and then added, or an introduction reagent and the nucleic acid can be separately added. The total amount of the introduction reagent and the nucleic acid can also be added at one time, or can also be divided into a plurality of doses for the addition.

The time of introducing the nucleic acid refers to the time of adding an introduction reagent (transfection reagent) for introduction of the nucleic acid in a case where the introduction reagent is used, or refers to the time of loading a current, a voltage, or the like in the case of loading of the current, a voltage, or the like for the introduction of the nucleic acid.

Additionally, the time of adding an introduction reagent refers to the first time of addition in a case where the introduction reagent is divided into multiple doses for the addition.

The lower limit of the final concentration of the cell growth inhibitor a described above is not particularly limited, can be selected as appropriate depending on the purpose, and is preferably 1 nM or more, and furthermore, more preferably 10 nM or more, 100 nM or more, 200 nM or more, 250 nM or more, 300 nM or more, or 400 nM or more in this order of preference, from the viewpoint of improvement of the effect of inducing viral vector production.

The upper limit of the final concentration of the cell growth inhibitor a described above is not particularly limited, can be selected as appropriate depending on the purpose, and is preferably 100 µM or less, and furthermore, more preferably 10 µM or less, 5 µM or less, 2.5 µM or less, 1 µM or less, 800 nM or less, or 600 nM or less in this order of preference, from the viewpoint of improvement of the effect of inducing viral vector production.

When the cell growth inhibitor a is nocodazole, the final concentration of nocodazole is not particularly limited, can be selected as appropriate depending on the purpose, and is preferably 100 nM or more and 5 µM or less, more preferably 250 nM or more and 2 µM or less, further preferably 300 nM or more and 1 µM or less, particularly preferably 400 nM or more and 800 nM or less, from the viewpoint of improvement of the effect of inducing viral vector production.

When the cell growth inhibitor a is albendazole, the final concentration of albendazole is not particularly limited, and can be selected as appropriate depending on the purpose, and is preferably 500 nM or more and 20 µM or less, more preferably 1 µM or more and 10 µM or less, from the viewpoint of improvement of the effect of inducing viral vector production.

When the cell growth inhibitor a is oxibendazole, the final concentration of oxibendazole is not particularly limited, and can be selected as appropriate depending on the purpose, and is preferably 500 nM or more and 200 µM or less, more preferably 1 µM or more and 150 µM or less, further preferably 10 µM or more and 100 µM or less, from the viewpoint of improvement of the effect of inducing viral vector production.

When the cell growth inhibitor a is mebendazole, the final concentration of mebendazole is not particularly limited, and can be selected as appropriate depending on the purpose, and is preferably 500 nM or more and 200 µM or less, more preferably 1 µM or more and 150 µM or less, further preferably 10 µM or more and 100 µM or less, from the viewpoint of improvement of the effect of inducing viral vector production.

When the cell growth inhibitor a is vinblastine, the final concentration of vinblastine is not particularly limited, and can be selected as appropriate depending on the purpose, and is preferably 10 nM or more and 10 µM or less, more preferably 50 nM or more and 10 µM or less, further preferably 100 nM or more and 5 µM or less, from the viewpoint of improvement of the effect of inducing viral vector production.

When the cell growth inhibitor a is colcemid, the final concentration of colcemid is not particularly limited, and can be selected as appropriate depending on the purpose, and is preferably 10 nM or more and 10 µM or less, more preferably 50 nM or more and 10 µM or less, further preferably 100 nM or more and 5 µM or less, from the viewpoint of improvement of the effect of inducing viral vector production.

The amount of a viral vector obtained by the method of producing a viral vector 1 is not particularly limited, and can be selected as appropriate depending on the purpose, and the titer of the viral vector relative to the titer of a viral vector in the case where the agent for inducing viral vector production is not used is preferably 1.1-fold or more, more preferably 1.2-fold or more, further preferably 1.5-fold or more, still further preferably 2-fold or more, and a higher ratio is more preferable.

The titer of the viral vector is determined by measurement according to a quantitative PCR method (Quantstudio3, SYBR Green method), as follows.

In the quantitative PCR, a material consisting of 12.5 µL of PowerUp^{™} SYBR^{®} Green Master Mix (Thermo Fisher Scientific K.K.), 0.125 µL of a forward primer (50 µM, SEQ ID NO: 1), 0.125 µL of a reverse primer (50 µM, SEQ ID NO: 2), 11.25 µL of Milli-Q^{®} water, and 1 µL of a reference product or a sample diluted 5000-fold (25 µL in total) is used.

PCR reaction for the above material is carried out by repeating 94°C/15 seconds (heat denaturation), 60°C/30 seconds (annealing), and 72°C/30 seconds (elongation reaction) for 30 cycles using QuantStudio3 (Thermo Fisher Scientific K.K.).

When the viral vector is the adeno-associated virus (AAV) vector, the above reference product to be used is those obtained by subjecting pAAV-MCS Expression Vector (0.67 µg/µL, TE solution, Cell Biolabs, Inc.) to digestion and linearization with PvuII (Takara Bio Inc.) at 37°C for 2 hours.

The titer of the viral vector in the case where the agent for inducing viral vector production is not used is not particularly limited, and can be selected as appropriate depending on the purpose, and is preferably 1 × 10¹³ vector genomes/L or more, more preferably 2 × 10¹³ vector genomes/L or more, further preferably 5 × 10¹³ vector genomes/L or more, still further preferably 8 × 10¹³ vector genomes/L or more, particularly preferably 1 × 10¹⁴ vector genomes/L or more, from the viewpoint of improvement of the effect of inducing viral vector production.

### (Method of producing viral vector 2)

A second aspect of the method of producing a viral vector comprises a step of introducing (transfecting) a nucleic acid into a cell and a step of adding cell growth inhibitor A described below to said cell, and can further comprise an additional step.

<Step of introducing (transfecting) a nucleic acid into cells>

The cell is not particularly limited and can be selected as appropriate depending on the purpose, and examples thereof include, *e.g.,* a mammalian cell and an insect cell.

Among them, a mammalian cell is preferred.

The mammalian cell is not particularly limited and can be selected as appropriate depending on the purpose, and examples thereof include a human-derived cell, a mouse-derived cell, a hamster-derived cell, a rat-derived cell, a dog-derived cell, a monkey-derived cell, and a kangaroo-derived cell.

Among them, HEK293 cell, CHO cell, Hela cell, Vero cell, BHK cell, COS cell, MDCK cell, 3T3 cell, HepG2 cell, A549 cell, C2C12 cell, C6 cell, HT-1080 cell, Huh-7 cell, H9C2 cell, HCT116 cell, HT-29 cell, K562 cell, LNCaP cell, Jurkat cell, L6 cell, USO2 cell, or PC12 cell is preferred, and HEK293 cell is more preferred, from the viewpoint of the yield of the viral vector and safety of the viral vector produced.

The cell described above includes a cell line derived from a parent cell. For example, a HEK293 cell-derived HEK293T cell, a FreeStyle (trademark) 293F cell, or Viral Production Cells 2.0 can be used as the HEK293 cell.

The insect cell is not particularly limited and can be selected as appropriate depending on the purpose, and examples thereof include a moth-derived cell and a fly-derived cell.

Among them, an Sf-9 cell, an Sf-21 cell, a High five cell, a Tni cell, or an S2 cell is preferred.

The cell described above includes a cell line derived from a parent cell.

The method of culturing the cell is not particularly limited and can be selected as appropriate depending on the purpose, and any of adhesion culture and suspension culture can be used.

Among them, suspension culture is preferably used from the viewpoint of the yield per volume of a culture medium and ease of operation.

The "nucleic acid" described above can also be called "polynucleotide", and examples thereof include deoxyribonucleic acid (DNA) and ribonucleic acid (RNA), and DNA is preferred. The DNA may be a single strand, double strand, or triple strand, and a double-stranded DNA is preferred. The structure of said double-stranded DNA is not particularly limited and can be selected as appropriate depending on the purpose, and examples include *e.g.,* a circular plasmid DNA, a linear plasmid DNA, an open circular plasmid DNA, a circular DNA, a linear DNA, and a linear covalently closed DNA.

Among them, a circular plasmid DNA, a circular DNA, or a linear covalently closed DNA is preferred, and a linear covalently closed DNA is more preferred from the viewpoint of gene introduction efficiency.

The "linear covalently closed DNA" is a linear double-stranded DNA having an end the structure of which is closed in a hairpin configuration, and can also be abbreviated as "LCC DNA".

The method of introducing a nucleic acid is not particularly limited and can be selected as appropriate depending on the purpose, and examples thereof include a method using a cationic polymer such as polyethylenimine (PEI), a method using liposome, a method using calcium phosphate, a method using a branched organic compound such as a dendrimer, a method using diethylaminoethyl (DEAE)-dextran, an electroporation method, a particle gun method, a microinjection method, a magnetofection method, and a method using a viral vector.

Among them, a method using a cationic polymer is preferred from the viewpoint of a high introduction efficiency and considering that the reagent is inexpensive, and also considering that any special apparatus is not necessary.

### <Step of adding cell growth inhibitor A to cells>

The cell growth inhibitor A is not particularly limited and can be selected as appropriate depending on the purpose, and examples thereof include *e.g.,* a compound which inhibits progression of cell cycle. The compound may be used as a single kind alone or as a mixture of two or more kinds.

The cell growth inhibitor A does not include a compound which induces cell death, such as sodium valproate.

Examples of the compound which inhibits progression of cell cycle include, *e.g.,* a compound which inhibits progression of cell cycle in G0 phase, a compound which inhibits progression of cell cycle in G1 phase, a compound which inhibits progression of cell cycle in S phase, and a compound which inhibits progression of cell cycle in G2 phase or M phase (corresponding to the cell growth inhibitor a described above).

Among them, a compound which inhibits progression of cell cycle in G2 phase or M phase is preferred from the viewpoint of improvement of the effect of inducing viral vector production.

Said compound which inhibits progression of cell cycle in G2 phase or M phase is as described above (agent for inducing viral vector production).

In the step of adding cell growth inhibitor A to a cell, an additional component can be added together with the cell growth inhibitor A. The additional component is as described above (agent for inducing viral vector production).

The timing of addition of the cell growth inhibitor A is not particularly limited as long as it is between 6 hours before and 6 hours after the time of introducing the nucleic acid, and the timing can be selected as appropriate depending on the purpose, and is more preferably between 4 hours before and 4 hours after the time of introducing the nucleic acid, further preferably between 5 minutes before and 4 hours after the time of introducing the nucleic acid, still further preferably between 5 minutes before and 2 hours after the time of introducing the nucleic acid, particularly preferably between the same time as and 2 hours after the time of introducing the nucleic acid, from the viewpoint of convenience, and improvement of the effect of inducing viral vector production.

The number of additions of the cell growth inhibitor A is not particularly limited, and can be selected as appropriate depending on the purpose. For example, the total amount can also be added at one time, or can also be divided into a plurality of doses for the addition.

The method of introducing the nucleic acid is not particularly restricted, and a complex of an introduction reagent (transfection reagent) and the nucleic acid can be formed and then added, or an introduction reagent and the nucleic acid can also be separately added. Additionally, the total amount of the introduction reagent and the nucleic acid can also be added at one time, or can also be divided into a plurality of doses for the addition.

The time of introducing the nucleic acid refers to the time of adding an introduction reagent (transfection reagent) for introduction of the nucleic acid in a case where the introduction reagent is used, or refers to the time of loading a current, a voltage, or the like in the case of loading of the current, a voltage, or the like for the introduction of the nucleic acid.

Additionally, the time of adding an introduction reagent refers to the first time of addition in a case where the introduction reagent is divided into a plurality of doses for the addition.

The lower limit of the final concentration of the cell growth inhibitor A is not particularly limited, and can be selected as appropriate depending on the purpose, and is preferably 1 nM or more, and furthermore, more preferably 10 nM or more, 100 nM or more, 200 nM or more, 250 nM or more, 300 nM or more, or 400 nM or more in this order of preference, from the viewpoint of improvement of the effect of inducing viral vector production.

The upper limit of the final concentration of the cell growth inhibitor A is not particularly limited, and can be appropriately selected depending on the purpose, and is preferably 100 µM or less, and furthermore, more preferably 10 µM or less, 5 µM or less, 2.5 µM or less, 1 µM or less, 800 nM or less, or 600 nM or less in this order of preference, from the viewpoint of improvement of the effect of inducing viral vector production.

When the cell growth inhibitor A is nocodazole, the final concentration of nocodazole is not particularly limited, and can be appropriately selected depending on the purpose, and is preferably 100 nM or more and 5 µM or less, more preferably 250 nM or more and 2 µM or less, further preferably 300 nM or more and 1 µM or less, particularly preferably 400 nM or more and 800 nM or less from the viewpoint of improvement of the effect of inducing viral vector production.

When the cell growth inhibitor A is albendazole, the final concentration of albendazole is not particularly limited, and can be appropriately selected depending on the purpose, and is preferably 500 nM or more and 20 µM or less, more preferably 1 µM or more and 10 µM or less, from the viewpoint of improvement of the effect of inducing viral vector production.

When the cell growth inhibitor A is oxibendazole, the final concentration of oxibendazole is not particularly limited, and can be appropriately selected depending on the purpose, and is preferably 500 nM or more and 200 µM or less, more preferably 1 µM or more and 150 µM or less, further preferably 10 µM or more and 100 µM or less, from the viewpoint of the improved effect of inducing viral vector production.

When the cell growth inhibitor A is mebendazole, the final concentration of mebendazole is not particularly limited, and can be appropriately selected depending on the purpose, and is preferably 500 nM or more and 200 µM or less, more preferably 1 µM or more and 150 µM or less, further preferably 10 µM or more and 100 µM or less, from the viewpoint of the improved effect of inducing viral vector production.

When the cell growth inhibitor A is vinblastine, the final concentration of vinblastine is not particularly limited, and can be appropriately selected depending on the purpose, and is preferably 10 nM or more and 10 µM or less, more preferably 50 nM or more and 10 µM or less, further preferably 100 nM or more and 5 µM or less, from the viewpoint of improvement of the effect of inducing viral vector production.

When the cell growth inhibitor A is colcemid, the final concentration of colcemid is not particularly limited, and can be appropriately selected depending on the purpose, and is preferably 10 nM or more and 10 µM or less, more preferably 50 nM or more and 10 µM or less, further preferably 100 nM or more and 5 µM or less, from the viewpoint of improvement of the effect of inducing viral vector production.

### <Additional step>

The additional step is not particularly limited and can be appropriately selected depending on the purpose, and examples thereof include, *e.g.,* a cell culture step before the step of introducing a nucleic acid into a cell, and a cell culture step after the step of adding cell growth inhibitor A to a cell.

### - Cell culture step before the step of introducing a nucleic acid into a cell -

The cell culture step before the step of introducing a nucleic acid into a cell is not particularly limited and can be appropriately selected depending on the purpose, and examples thereof include, e.g., a method of culturing a cell at 37°C in the presence of 8% CO₂ and adjusting the cell number before seeding in a culture vessel.

The cell number to be seeded in the culture vessel is not particularly limited, and can be appropriately selected depending on the purpose, and is preferably 1 × 10⁵ cells/mL or more and 1 × 10⁹ cells/mL or less, more preferably 3 × 10⁵ cells/mL or more and 1 × 10⁸ cells/mL or less, further preferably 5 × 10⁵ cells/mL or more and 1 × 10⁷ cells/mL or less, from the viewpoint of improvement of the effect of inducing viral vector production.

The volume of culture medium for seeding in the culture vessel is not particularly limited, and can be appropriately selected depending on the purpose, and is preferably 0.1 mL or more and 1000 L or less, more preferably 1 mL or more and 100 L or less, further preferably 3 mL or more and 10 L or less, particularly preferably 5 mL or more and 1 L or less, from the viewpoint of improvement of the effect of inducing viral vector production.

### - Cell culture step after the step of adding cell growth inhibitor A to a cell -

The cell culture step after the step of adding cell growth inhibitor A to a cell is not particularly limited and can be appropriately selected depending on the purpose, and examples thereof include, *e.g.,* a method of culturing the cell after the addition of the cell growth inhibitor A, at 37°C in the presence of 8% CO₂, and extracting the viral vector from the cell culture containing the cell after the culturing.

The number of days of the culturing is not particularly limited, and can be appropriately selected depending on the purpose, and is preferably 1 day or more and 7 days or less, more preferably 2 days or more and 6 days or less, further preferably 3 days or more and 5 days or less, from the viewpoint of improvement of the effect of inducing viral vector production.

The amount of the viral vector obtained by the method of producing a viral vector 2 is not particularly limited, and can be appropriately selected depending on the purpose, and the titer of the viral vector relative to the titer of the viral vector in the case where the cell growth inhibitor A is not added is preferably 1.1-fold or more, more preferably 1.2-fold or more, further preferably 1.5-fold or more, still further preferably 2-fold or more, and a higher value is more preferable.

The titer of the viral vector is determined by measurement according to quantitative PCR methods.

The quantitative PCR method is as described above (method of producing a viral vector 1).

The titer of the viral vector in the case where the cell growth inhibitor A is not used is not particularly limited, and can be appropriately selected depending on the purpose, and is preferably 1 × 10¹³ vector genomes/L or more, more preferably 2 × 10¹³ vector genomes/L or more, further preferably 5 × 10¹³ vector genomes/L or more, still further preferably 8 × 10¹³ vector genomes/L or more, particularly preferably 1 × 10¹⁴ vector genomes/L or more, from the viewpoint of improvement of the effect of inducing viral vector production.

### (Method of inducing viral vector production 1)

A first aspect of the method of inducing viral vector production is a method using the agent for inducing viral vector production described above comprising cell growth inhibitor a.

The amount of the viral vector obtained by the method of inducing viral vector production 1 is not particularly limited and can be appropriately selected depending on the purpose, and the titer of the viral vector relative to the titer of a viral vector in the case where the agent for inducing viral vector production is not used is preferably 1.1-fold or more, more preferably 1.2-fold or more, further preferably 1.5-fold or more, still further preferably 2-fold or more, and a higher value is more preferable.

The titer of the viral vector is determined by measurement according to quantitative PCR methods.

The quantitative PCR method is as described above (method of producing a viral vector 1).

The titer of the viral vector in the case where the agent for inducing viral vector production is not used is not particularly limited, and can be appropriately selected depending on the purpose, and is preferably 1 × 10¹³ vector genomes/L or more, more preferably 2 × 10¹³ vector genomes/L or more, further preferably 5 × 10¹³ vector genomes/L or more, still further preferably 8 × 10¹³ vector genomes/L or more, particularly preferably 1 × 10¹⁴ vector genomes/L or more, from the viewpoint of improvement of the effect of inducing viral vector production.

The method using the agent for inducing viral vector production is as described above (method of producing a viral vector 1).

### (Method of inducing viral vector production 2)

A second aspect of the induction of viral vector production comprises a step of introducing a nucleic acid into a cell, and a step of adding cell growth inhibitor A to the cell, and can further comprise an additional step.

The step of introducing a nucleic acid into a cell, the step of adding cell growth inhibitor A to said cell, and the additional step are as described above (method of producing a viral vector 2).

The amount of the viral vector obtained by the method of inducing viral vector production 2 is not particularly limited and can be appropriately selected depending on the purpose, and the titer of the viral vector relative to the titer of a viral vector in the case where the cell growth inhibitor A is not added is preferably 1.1-fold or more, more preferably 1.2-fold or more, further preferably 1.5-fold or more, still further preferably 2-fold or more, and a higher value is more preferable.

The titer of the viral vector is determined by measurement according to a quantitative PCR method.

The quantitative PCR method is as described above (method of producing a viral vector 1).

The titer of the viral vector in the case where the cell growth inhibitor A is not used is not particularly limited, and can be appropriately selected depending on the purpose, and is preferably 1 × 10¹³ vector genomes/L or more, more preferably 2 × 10¹³ vector genomes/L or more, further preferably 5 × 10¹³ vector genomes/L or more, still further preferably 8 × 10¹³ vector genomes/L or more, particularly preferably 1 × 10¹⁴ vector genomes/L or more, from the viewpoint of improvement of the effect of inducing viral vector production.

### Examples

Hereinafter, Examples of the present invention are described, but the present invention is not limited to these Examples at all.

### <Cell culture>

A suspension HEK293 cell (Thermo Fisher Scientific K.K.; FreeStyle^{™} 293F cell) was seeded in a culture vessel, and cultured by passage every three days or four days at 37°C in the presence of 8% CO₂.

### <Nucleic acid>

### <<Production Example 1: Double-stranded circular plasmid DNA>>

pRC2-mi342 (packaging plasmid DNA) and pHelper (helper plasmid DNA) were purchased from Takara Bio Inc. (AAVpro^{®} Helper Free System). pAAV-Venus (vector plasmid DNA) was manufactured by substituting a nucleic acid sequence encoding a green fluorescent protein (GFP) of pAAV-GFP Control Plasmid (Cell Biolabs, Inc.), with a nucleic acid sequence (SEQ ID NO: 3) encoding a fluorescent protein (Venus).

### <<Production Example 2: Linear covalently closed DNA>>

RC2_LCC_DNA, Helper_LCC_DNA and Venus_LCC_DNA were produced as follows.

In Production Examples 2-2, 2-3, and 2-4 below, a double-stranded circular plasmid DNA used for transformation of *E. coli* was prepared by introducing a constructed vector into an *E. coli* DH5α competent cell (9057, Takara Bio Inc.), and culturing and amplifying the resulting transformant. Preparation of a plasmid from a strain retaining the double-stranded circular plasmid DNA was performed with FastGene Plasmid Mini Kit (NIPPON Genetics Co, Ltd.).

### <Production Example 2-1: Preparation of various genes used for preparation of vector>

The nucleic acid sequence (SEQ ID NO: 4) encoding Escherichia virus N15-derived protelomerase (TelN protelomerase), used in the vector construction, was prepared by PCR with a synthetic DNA as a template. The synthetic DNA can be produced by, *e.g.,* a method of synthesizing a plurality of oligonucleotides designed such that sequences are overlapped, and annealing the oligonucleotides and then elongating them with DNA ligase or DNA polymerase, or can also be obtained by use of the artificial gene synthesis service provided by each company.

The nucleic acid fragment (SEQ ID NO: 5) was totally synthesized as the paired nucleic acid sequences (telRL sequences) which are recognized by protelomerase, and was used in the vector construction.

The AraC gene (SEQ ID NO: 6) controlled by a promoter, which was used in the vector construction, was prepared by PCR using a synthetic DNA as a template. The synthetic DNA can be produced by, *e.g.,* a method of synthesizing a plurality of oligonucleotides designed such that sequences are overlapped, and annealing the oligonucleotides and then elongating them with DNA ligase or DNA polymerase, or can also be obtained by using the artificial gene synthesis service provided by each company.

The nucleic acid fragment (SEQ ID NO: 7) was totally synthesized as an arabinose inducible promoter which was used in the vector construction.

The nucleic acid sequence (SEQ ID NO: 3) encoding a fluorescent protein (Venus), which was used in the vector construction, was prepared by PCR using a synthetic DNA as a template. The synthetic DNA can be produced by, *e.g.,* a method of synthesizing a plurality of oligonucleotides designed such that sequences are overlapped, and annealing the oligonucleotides and then elongating them with DNA ligase or DNA polymerase, or can also be obtained by using the artificial gene synthesis service provided by each company.

The nucleic acid sequence encoding TelN protelomerase was prepared by PCR using Primer 1 (SEQ ID NO: 8, forward primer) and Primer 2 (SEQ ID NO: 9, reverse primer), an AraC gene controlled by a promoter was prepared by PCR using Primer 3 (SEQ ID NO: 10, forward primer) and Primer 4 (SEQ ID NO: 11, reverse primer), and a nucleic acid sequence encoding a fluorescent protein (Venus) was prepared by PCR using Primer 5 (SEQ ID NO: 12, forward primer) and Primer 6 (SEQ ID NO: 13, reverse primer).

Prime STAR MAX DNA Polymerase (Takara Bio Inc.) was used in the PCR, and reaction conditions were in accordance with the method described in the manual attached.

### <Production Example 2-2: Construction of the vector having the gene of interest>

The nucleic acid fragment was prepared by PCR for the nucleic acid sequence (SEQ ID NO: 3) encoding a fluorescent protein (Venus) with Primer 5 (SEQ ID NO: 12) and Primer 6 (SEQ ID NO: 13), and was substituted with the nucleic acid sequence encoding a green fluorescent protein (GFP) of pAAV-GFP Control Plasmid (Cell Biolabs, Inc.) for constructing pAAV-Venus.

Then, the nucleic acid fragment (SEQ ID NO: 5) of a pair of nucleic acid sequences (telRL sequences) recognized by protelomerase was prepared by total synthesis, and inserted into PciI site and KasI site of pAAV-Venus for constructing pAAV-Venus_telRL.

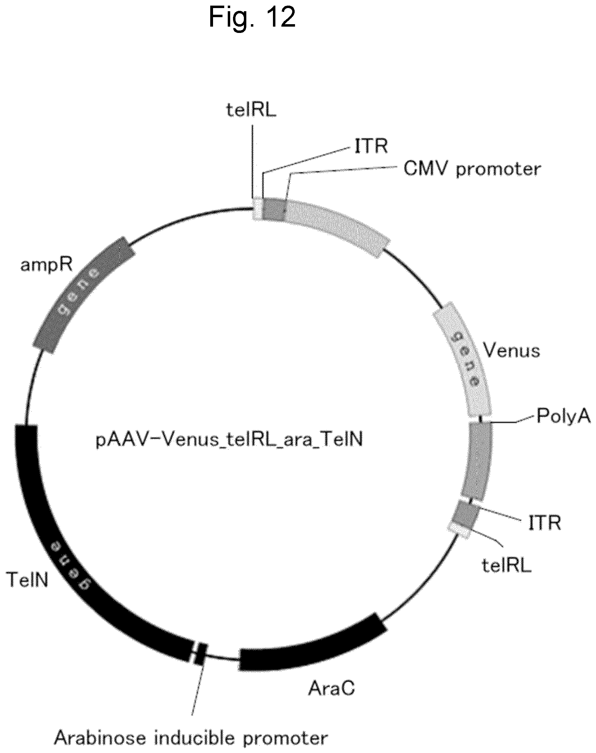

Next, a nucleic acid fragment was prepared by PCR the nucleic acid sequence (SEQ ID NO: 4) encoding TelN protelomerase with Primer 1 (SEQ ID NO: 8) and Primer 2 (SEQ ID NO: 9), and a nucleic acid fragment was prepared by PCR for the AraC gene (SEQ ID NO: 6) under the control of a promoter, with Primer 3 (SEQ ID NO: 10) and Primer 4 (SEQ ID NO: 11), and the nucleic acid fragment (SEQ ID NO: 7) of an arabinose inducible promoter was prepared by total synthesis, and each was inserted into PsiI site of pAAV-Venus_telRL for constructing pAAV-Venus_telRL_ara_TelN (Figure 12).

The pAAV-Venus_telRL_ara_TelN vector is a vector for production of a linear covalently closed DNA, and is designed so that TelN protelomerase is expressed under the control of an arabinose inducible promoter. In addition, telRL sequences are used as a pair of nucleic acid sequences recognized by the protelomerase, and a nucleic acid sequence encoding a fluorescent protein (Venus) is located between the telRL sequences as a nucleic acid sequence (gene of interest) encoding a protein of interest.

The telRL in Figure 12 is a pair of nucleic acid sequences (telRL sequences) recognized by protelomerase. The ITR in Figure 12 is an inverted terminal repeat. The CMV Promotor in Figure 12 is a cytomegalovirus-derived promoter. The Venus in Figure 12 is a nucleic acid sequence encoding a fluorescent protein (Venus). The PolyA in Figure 12 is a sequence involved in polyadenylation of messenger RNA. The AraC in Figure 12 is an AraC gene sequence under the control of a promoter. The Arabinose inducible Promoter in Figure 12 is a nucleic acid sequence of an arabinose inducible promoter. The TelN in Figure 12 is a nucleic acid sequence encoding TelN protelomerase. The ampR in Figure 12 is a nucleic acid sequence encoding an antibiotic-resistant protein (AmpR).

### <Production Example 2-3: Construction of the vector having a packaging gene>

The nucleic acid fragment (SEQ ID NO: 5) of a pair of nucleic acid sequences (telRL sequences) recognized by protelomerase was prepared by total synthesis, and inserted into EcoRV site and SnaBI site of pRC2-mi342 (Takara Bio Inc.) for constructing pRC2-mi342_telRL.

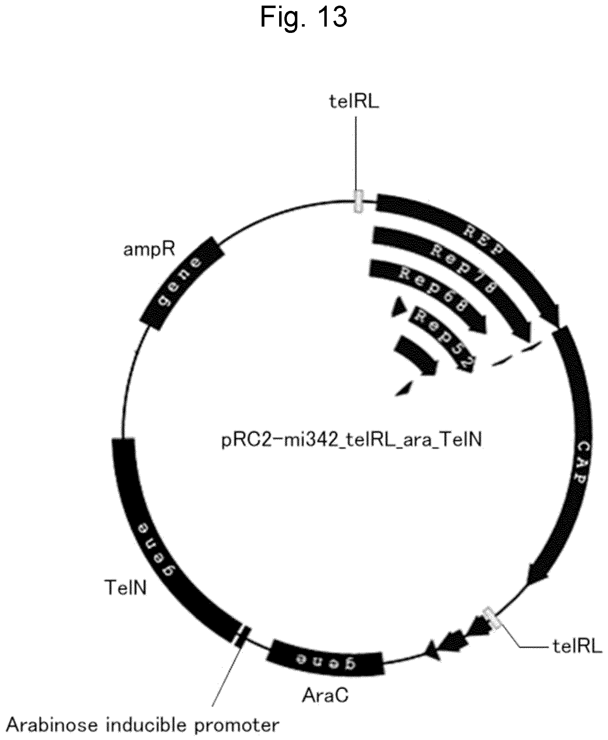

Next, a nucleic acid fragment was prepared by PCR for the nucleic acid sequence (SEQ ID NO: 4) encoding TelN protelomerase, with Primer 1 (SEQ ID NO: 8) and Primer 2 (SEQ ID NO: 9), and a nucleic acid fragment was prepared by PCR for the AraC gene (SEQ ID NO: 6) under the control of a promoter, with Primer 3 (SEQ ID NO: 10) and Primer 4 (SEQ ID NO: 11), and a nucleic acid fragment (SEQ ID NO: 7) of an arabinose inducible promoter was prepared by total synthesis, and each was inserted into SmaI site of pRC2-mi342_telRL for constructing pRC2-mi342_telRL_ara_TelN (Figure 13).

The pRC2-mi342_telRL_ara_TelN vector is a vector for the production of a linear covalently closed DNA, and is designed such that TelN protelomerase is expressed under the control of an arabinose inducible promoter. In addition, telRL sequences are used as a pair of nucleic acid sequences recognized by protelomerase, and the nucleic acid sequences encoding packaging proteins (Rep: SEQ ID NO: 14, Cap: SEQ ID NO: 15) are located between the telRL sequences.

The Rep in Figure 13 is a nucleic acid sequence encoding the packaging protein Rep of an adeno-associated virus. The Cap in Figure 13 is a nucleic acid sequence encoding the packaging protein Cap of an adeno-associated virus.

### <Production Example 2-4: Construction of the vector having a helper gene>

The nucleic acid fragment (SEQ ID NO: 5) of a pair of nucleic acid sequences (telRL sequences) recognized by protelomerase was prepared by total synthesis, and inserted into BamHI site and SalI site of pHelper (Takara Bio Inc.) for constructing pHelper_telRL.

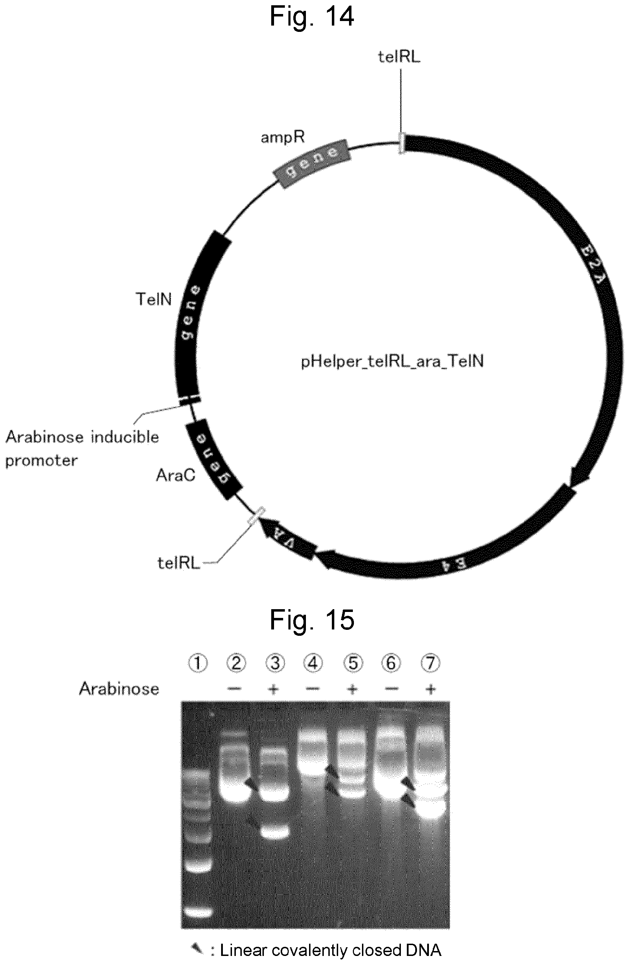

Next, a nucleic acid fragment was prepared by PCR for the nucleic acid sequence (SEQ ID NO: 4) encoding TelN protelomerase, with Primer 1 (SEQ ID NO: 8) and Primer 2 (SEQ ID NO: 9), and a nucleic acid fragment was prepared by PCR for the AraC gene (SEQ ID NO: 6) under the control of a promoter, with Primer 3 (SEQ ID NO: 10) and Primer 4 (SEQ ID NO: 11), and the nucleic acid fragment (SEQ ID NO: 7) of an arabinose inducible promoter was prepared by total synthesis, and each was inserted into NdeI site of pHelper_telRL for constructing pHelper_telRL_ara_TelN (Figure 14).

The pHelper_telRL_ara_TelN vector is a vector for the production of a linear covalently closed DNA, and is designed such that TelN protelomerase is expressed under the control of an arabinose inducible promoter. In addition, telRL sequences are used as a pair of nucleic acid sequences recognized by protelomerase, and the nucleic acid sequences encoding helper proteins (E2A: SEQ ID NO: 16, E4: SEQ ID NO: 17, VA: SEQ ID NO: 18) are located between the telRL sequences.

The E2A in Figure 14 is a nucleic acid sequence encoding the helper protein E2A of an adenovirus. The E4 in Figure 14 is a nucleic acid sequence encoding the helper protein E4 of an adenovirus. The VA in Figure 14 is a nucleic acid sequence encoding the helper protein VA of an adenovirus.

### <Production Example 2-5: Transformation of E. coli>

The vector for the production of a linear covalently closed DNA which was constructed in Production Example 2-2, pAAV-Venus_telRL_ara_TelN, the vector for the production of a linear covalently closed DNA which was constructed in Production Example 2-3, pRC2-mi342_telRL_ara_TelN, or the vector for the production of a linear covalently closed DNA which was constructed in Production Example 2-4, pHelper_telRL_ara_TelN, was used for transformation of *E. coli* as described below.

Twenty-five microliter of a solution of a competent cell of an *E. coli* NEB10β strain (New England Biolabs, Inc., C3019H) was mixed with a solution of the vector for the production of a linear covalently closed DNA which comprised 100 pg of the pAAV-Venus_telRL_ara_TelN, 100 pg of the pRC2-mi342_telRL_ara_TelN, or 100 pg of the pHelper_telRL_ara_TelN, and the mixture was left to stand on ice for 30 minutes.

The mixture was left to stand for 30 minutes, thereafter heat-treated at 42°C for 45 seconds, and left to stand on ice for 2 minutes.

The mixture was left to stand for 2 minutes, 225 µL of an SOC culture medium was added thereto, and *E. coli* was plated to an LB agar culture medium (1% tryptone, 0.5% dry yeast extract, 1% sodium chloride, 0.005% carbenicillin disodium (NACALAI TESQUE, INC.)), and a strain to be grown was selected by static culture at 37°C for 1 day, and *E. coli* to which the vector for the production of a linear covalently closed DNA was introduced was obtained.

### <Production Example 2-6: Culture of transformed E. coli>

The transformed *E. coli* obtained in Production Example 2-5 was seeded to 2 mL of Plusgrow II culture medium (4% Plusgrow II (NACALAI TESQUE, INC.), 0.005% carbenicillin disodium), and cultured with shaking at 37°C for 6 hours, and then a pre-culture medium was obtained.

One hundred microliter of the pre-culture medium was subjected to subculture in 50 mL of Plusgrow II culture medium (4% Plusgrow II, 0.005% carbenicillin disodium), and was cultured with shaking at 37°C for 16 hours.

After culturing with shaking for 16 hours, 50 mL of Plusgrow II culture medium (4% Plusgrow II, 0.005% carbenicillin disodium) and 1 mL of an arabinose solution (10% arabinose) were added, followed by culturing with shaking at 37°C for 1 hour. After culturing with shaking for 1 hour, the bacterial cells were collected by centrifugation.

### <<Evaluation of the linear covalently closed DNA by electrophoresis>>

The linear covalently closed DNA prepared from the *E. coli* bacteria cell obtained in Production Example 2-6 was evaluated by electrophoresis as described below.

Preparation of DNA from the *E. coli* bacteria cells was performed using FastGene Plasmid Mini Kit (NIPPON Genetics Co, Ltd.). The resulting DNA solution was applied to wells of 1% agarose gel prepared with TAE buffer, and electrophoresed at 100 V for 40 minutes. Staining was performed with a nucleic acid staining agent, and UV light was used for detection. The results are shown in lanes 3, 5, and 7 in Figure 15.

The evaluation results of DNA obtained from *E. coli* bacteria cells cultured in the same manner except that 1 mL of the arabinose solution was not added are shown in lanes 2, 4, and 6 in Figure 15.

In Figure 15, lane 1 represents DNA marker (1kb DNA Ladder, 3412A, Takara Bio Inc.), lane 3 represents pAAV-Venus_telRL_ara_TelN-derived sample, lane 5 represents pHelper_telRL_ara_TelN-derived sample, lane 7 represents pRC2-mi342_telRL_ara_TelN-derived sample, lane 2 represents pAAV-Venus_telRL_ara_TelN-derived sample without addition of arabinose solution, lane 4 represents pHelper_telRL_ara_TelN-derived sample without addition of arabinose solution, and lane 6 represents pRC2-mi342_telRL_ara_TelN-derived sample without addition of arabinose solution.

In the results of Figure 15, two bands derived from the linear covalently closed DNA (as indicated by arrows in Figure 15, a band derived from a linear covalently closed DNA having the nucleic acid sequence encoding the protein of interest, a band derived from the linear covalently closed DNA having the nucleic acid sequence encoding the packaging protein, or a band derived from the linear covalently closed DNA having the nucleic acid sequence encoding the helper protein, and a band derived from the linear covalently closed DNA having the nucleic acid sequence encoding TelN protelomerase) were observed in the *E. coli* bacteria cells derived from the culture medium with addition of arabinose (lanes 3, 5, and 7), whereas a linear covalently closed DNA-derived band was not observed and only a double-stranded circular plasmid DNA-derived band was observed in the *E. coli* bacteria cells derived from the culture medium without addition of arabinose (lanes 2, 4, and 6).

These results suggested that when the vector having the nucleic acid sequence encoding protelomerase, the pair of nucleic acid sequences recognized by protelomerase, and the nucleic acid sequence located between the pair of nucleic acid sequences and encoding the protein was introduced into *E. coli,* TelN protelomerase cleaved and recombined the telRL sequence recognized by TelN protelomerase, and a linear covalently closed DNA having the nucleic acid sequence encoding the protein of interest located between telRL sequences, the nucleic acid sequence encoding the packaging protein located between telRL sequences, or the nucleic acid sequence encoding the helper protein located between telRL sequences was produced.

### <Production Example 2-7: Purification of the linear covalently closed DNA>

The linear covalently closed DNA obtained in Production Example 2-6 was treated with the restriction enzyme BstZ17I (New England Biolabs, Inc.), and the linear covalently closed DNA was purified with NucleoSpin Gel and PCR Clean-up Kit (MACHEREY-NAGEL GmbH & Co. KG). Thus, a linear covalently closed DNA having a nucleic acid sequence encoding TelN protelomerase and a vector for the production of a linear covalently closed DNA were cleaved at the BstZ17I recognition site thereof.

Next, treatment with Exonuclease (New England Biolabs, Inc.) was performed, and the linear covalently closed DNA was purified with NucleoSpin Gel and PCR Clean-up Kit (MACHEREY-NAGEL GmbH & Co. KG). Thus, the nucleic acid was degraded to nucleotides from the end of the single-stranded or double-stranded DNA.

### <<Evaluation of linear covalently closed DNA by electrophoresis>>

The linear covalently closed DNA obtained in Production Example 2-7 was evaluated by electrophoresis, according to the method described below.

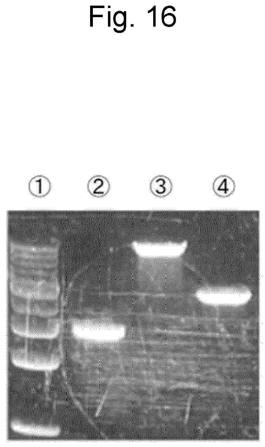

The resulting DNA solution was applied to the wells of 1% agarose gel prepared with TAE buffer, and electrophoresed at 100 V for 40 minutes. Staining was performed with a nucleic acid staining agent, and UV light was used for detection. The results are shown in Figure 16 (lanes 2, 3, and 4).

Herein, a linear covalently closed DNA having a nucleic acid sequence encoding a protein of interest located between telRL sequences is referred to as Venus_LCC_DNA, a linear covalently closed DNA having a nucleic acid sequence encoding a packaging protein located between telRL sequences is referred to as RC2_LCC_DNA, and a linear covalently closed DNA having a nucleic acid sequence encoding a helper protein located between telRL sequences is referred to as Helper_LCC_DNA.

Lane 1 in Figure 16 represents DNA marker (1kb DNA Ladder, Takara Bio Inc., 3412A), lane 2 in Figure 16 represents Venus_LCC_DNA, lane 3 in Figure 16 represents Helper_LCC_DNA, and lane 4 in Figure 16 represents RC2_LCC_DNA.

In the results of Figure 16, a band derived from the linear covalently closed DNA having the nucleic acid sequence encoding the protein located between telRL sequences was observed, whereas bands derived from the linear covalently closed DNA having the nucleic acid sequence encoding TelN protelomerase and derived from the vector for the production of linear covalently closed DNA production were not observed.

These results suggested that the nucleic acid sequence encoding TelN protelomerase was cleaved by the restriction enzyme BstZ17I and the nucleic acid was degraded to nucleotides by Exonuclease from the end of single-stranded or double-stranded DNA. Thus, this result suggested that the ends of the linear covalently closed DNA obtained in Production Example 2-6 were certainly closed.

### <Example 1: Influence of the final concentration of the cell growth inhibitor A (containing the cell growth inhibitor a) on the titer of adeno-associated virus>

This Example 1 corresponds to all of the method of producing a viral vector 1, the method of producing a viral vector 2, the method of inducing viral vector production 1, and the method of inducing viral vector production 2.

On the day of introduction of the nucleic acid into cells, suspension HEK293 cells (Thermo Fisher Scientific K.K.; FreeStyle^{™} 293F cell) were seeded in a 50-mL centrifuge tube equipped with a filter cap, with the number of cells adjusted to 1 × 10⁶ cells/mL (an amount of culture medium of 5 mL).

pRC2-mi342 (1 µg/µL, 3 µL, packaging plasmid DNA), pAAV-Venus (1 µg/µL, 1 µL, vector plasmid DNA), pHelper (1 µg/µL, 2 µL, helper plasmid DNA), and PEI MAX (1 µg/µL, 12 µL, Polysciences Inc.) as polyethylenimine (PEI) were mixed and left to stand in Opti-MEM (Thermo Fisher Scientific K.K.) for producing a double-stranded circular plasmid DNA-PEI mixed solution.

Nucleic acids were introduced into the cells by adding the double-stranded circular plasmid DNA-PEI mixed solution to the cell culture medium. Thereafter, AAV2 was produced by culture at 37°C in the presence of 8% CO₂ for 4 days. In addition to nocodazole (Cayman Chemical Company) as cell growth inhibitor A, DMSO as a solubilizer was used for adjustment to 0 nM, 25 µM, 50 µM, or 200 µM, and an amount (50 µL) corresponding to 1% relative to an amount of the cell culture medium of 5 mL was added 4 hours after the introduction of the nucleic acid into the cells so that the final concentration of nocodazole was 0 nM, 250 nM, 500 nM, or 2 µM.

Extraction of the adeno-associated virus from the cell culture medium was carried out as follows.

Four days after the introduction of the nucleic acid into cells, the supernatant and the cell were separated, PEG8000 (Sigma-Aldrich Co. LLC) was added to the supernatant obtained, and the adeno-associated virus vector was concentrated. Triton X-100 (Sigma-Aldrich Co. LLC) was added to the residual for cell lysis, and thereafter KANEKA Endonuclease (KANEKA CORPORATION) was added for degradation of the nucleic acid. EDTA (NIPPON GENE CO., LTD.) was added and centrifuged, and the adeno-associated virus vector was obtained (supernatant-derived sample). Triton X-100 (Sigma-Aldrich Co. LLC) was added to the resulting cell pellets for cell lysis, and thereafter KANEKA Endonuclease (KANEKA CORPORATION) was added for degradation of the nucleic acid. EDTA (NIPPON GENE CO., LTD.) was added and centrifugation was performed, and the adeno-associated virus vector was obtained (cell-derived sample).

Quantification of the adeno-associated virus vector was carried out as follows.

The titer of the adeno-associated virus vector contained in the supernatant-derived sample and the cell-derived sample was measured by a quantitative PCR method (QuantStudio3, SYBR-Green method) with the forward primer (SEQ ID NO: 1) and the reverse primer (SEQ ID NO: 2), as described below.

In the quantitative PCR, a material consisting of 12.5 µL of PowerUp^{™} SYBR^{®} Green Master Mix (A25742, Thermo Fisher Scientific K.K.), 0.125 µL of the forward primer (50 µM, SEQ ID NO: 1), 0.125 µL of the reverse primer (50 µM, SEQ ID NO: 2), 11.25 µL of Milli-Q^{®} water, and 1 µL of a reference product or a sample diluted 5000-fold (25 µL in total) was used.

PCR reaction for the above material was carried out by repeating 94°C/15 seconds (heat denaturation), 60°C/30 seconds (annealing), and 72°C/30 seconds (elongation reaction) for 30 cycles with QuantStudio3 (Thermo Fisher Scientific K.K.).

The above reference product was produced by digesting pAAV-MCS Expression Vector (0.67 µg/µL, TE solution, Cell Biolabs, Inc.) for linearization with PvuII (1243A, Takara Bio Inc.) at 37°C for 2 hours.

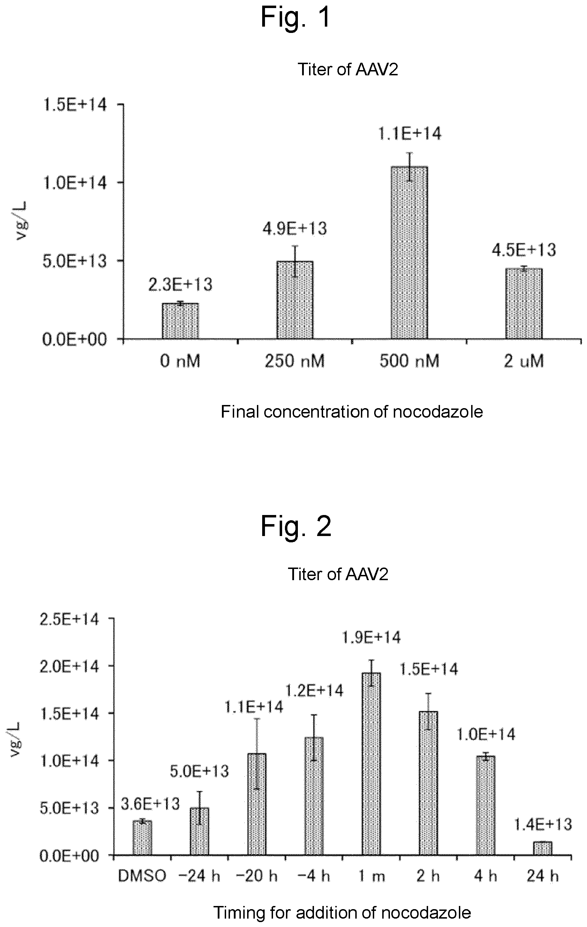

Figure 1 shows the influence of the final concentration of nocodazole as cell growth inhibitor A on the titer of the adeno-associated virus. Figure 1 shows the total titer of adeno-associated virus vectors contained in the cell-derived sample and the supernatant-derived sample. In Figure 1, "0 nM" indicates that the final concentration of nocodazole was 0 nM and that only 50 µL of DMSO was added 4 hours after introduction of the nucleic acid into the cells.

The results shown in Figure 1 indicated that the titer of the adeno-associated virus vector was improved by the addition of the cell culture medium so that the final concentration of nocodazole as cell growth inhibitor A was from 250 nM to 2 µM.

### <Example 2: Influence of timing of addition of the cell growth inhibitor A (containing the cell growth inhibitor a) on the titer of the adeno-associated virus>

This Example 2 corresponds to all of the method of producing a viral vector 1, the method of producing a viral vector 2, the method of inducing viral vector production 1, and the method of inducing viral vector production 2 (the method of producing a viral vector 2 and the method of inducing viral vector production 2 correspond to the cases of addition of 4 hours before introduction of cell growth inhibitor A into a cell, and 1 minute after, 2 hours after, or 4 hours after introduction of a nucleic acid into a cell).

Nucleic acids were introduced into the cells with the same method as in Example 1, and the adeno-associated virus vector was produced in the conditions where the timing of addition of cell growth inhibitor A was different, using a constant final concentration of cell growth inhibitor A, and the titer was measured. Nocodazole as cell growth inhibitor A and DMSO as a solubilizer were used for adjustment to 50 µM, and an amount (50 µL) corresponding to 1% relative to an amount of the cell culture medium of 5 mL was added 24 hours before, 20 hours before, and 4 hours before introduction of the nucleic acid into the cells, and 1 minute after, 2 hours after, 4 hours after, and 24 hours after introduction of the nucleic acid into the cells so that the final concentration of nocodazole was 500 nM.

Figure 2 shows the influence of the timing of addition of cell growth inhibitor A on the titer of the adeno-associated virus vector. Figure 2 shows the titer of the adeno-associated virus vector contained in the cell-derived sample. In Figure 2, "DMSO" shows that only 50 µL of DMSO was added 1 minute after introduction of the nucleic acid into the cells. "1m", "4h", and "-4h" respectively show that cell growth inhibitor A was added 1 minute after introduction of the nucleic acid into the cells, 4 hours after introduction of the nucleic acid into the cells, and 4 hours before introduction of the nucleic acid into the cells.

The results shown in Figure 2 indicated that the titer of the adeno-associated virus vector was improved by the addition of cell growth inhibitor A to the cell culture medium between 24 hours before and 4 hours after the introduction of the nucleic acid into the cells.

### <Example 3: Comparison of various cell growth inhibitors A (containing the cell growth inhibitor a)>

This Example 3 corresponds to all of the method of producing a viral vector 1, the method of producing a viral vector 2, the method of inducing viral vector production 1, and the method of inducing viral vector production 2.

Nucleic acids were introduced into the cells with the same procedure as in Example 1, the adeno-associated virus was produced in the condition where different types of cell growth inhibitor A and a solubilizer were added, and the titer was measured. Various cell growth inhibitors A were adjusted with the solubilizer to the 100-fold concentration of the intended final concentration in the same manner as in Example 1, and an amount (50 µL) corresponding to 1% relative to an amount of the cell culture medium of 5 mL was added 4 hours after introduction of the nucleic acid into the cells.

When the cell growth inhibitor A was nocodazole, DMSO was used as a solubilizer and the nocodazole was added to cell culture medium so that the final concentration of nocodazole was 500 nM. When the cell growth inhibitor A was fenbendazole (FUJIFILM Wako Pure Chemical Corporation), albendazole (Tokyo Chemical Industry Co., Ltd.), or mebendazole (Tokyo Chemical Industry Co., Ltd.), DMSO was used as a solubilizer and the cell growth inhibitor A was added to the cell culture medium so that the final concentrations of the cell growth inhibitor A were three kinds of concentration, *i.e.,* 50 µM, 5 µM, and 500 nM. When the cell growth inhibitor A was vinblastine sulfate (FUJIFILM Wako Pure Chemical Corporation), colcemid (Cayman Chemical Company), paclitaxel (FUJIFILM Wako Pure Chemical Corporation), or docetaxel (LKT Laboratories, Inc.), DMSO was used as a solubilizer and the cell growth inhibitor A was added to the cell culture medium so that the final concentrations of the cell growth inhibitor A were three kinds of concentrations, *i.e.,* 5 µM, 500 nM, and 100 nM. When the cell growth inhibitor A was aphidicolin (FUJIFILM Wako Pure Chemical Corporation) or MG-132 (FUJIFILM Wako Pure Chemical Corporation), DMSO was used as a solubilizer and the cell growth inhibitor A was added to the cell culture medium so that the final concentrations of the cell growth inhibitor A were three kinds of concentrations, *i.e.,* 100 nM, 10 nM, and 1 nM. When the cell growth inhibitor A was genistein (FUJIFILM Wako Pure Chemical Corporation) or roscovitine (FUJIFILM Wako Pure Chemical Corporation), DMSO was used as a solubilizer and the cell growth inhibitor A was added to the cell culture medium so that the final concentrations of the cell growth inhibitor A were three kinds of concentrations, *i.e.,* 10 µM, 500 nM, 100 nM. When the cell growth inhibitor A was thymidine (Tokyo Chemical Industry Co., Ltd.), water was used as a solubilizer and the cell growth inhibitor A was added to the cell culture medium so that the final concentrations of thymidine were three kinds of concentrations, *i.e.,* 10 mM, 1 mM, and 0.1 mM.

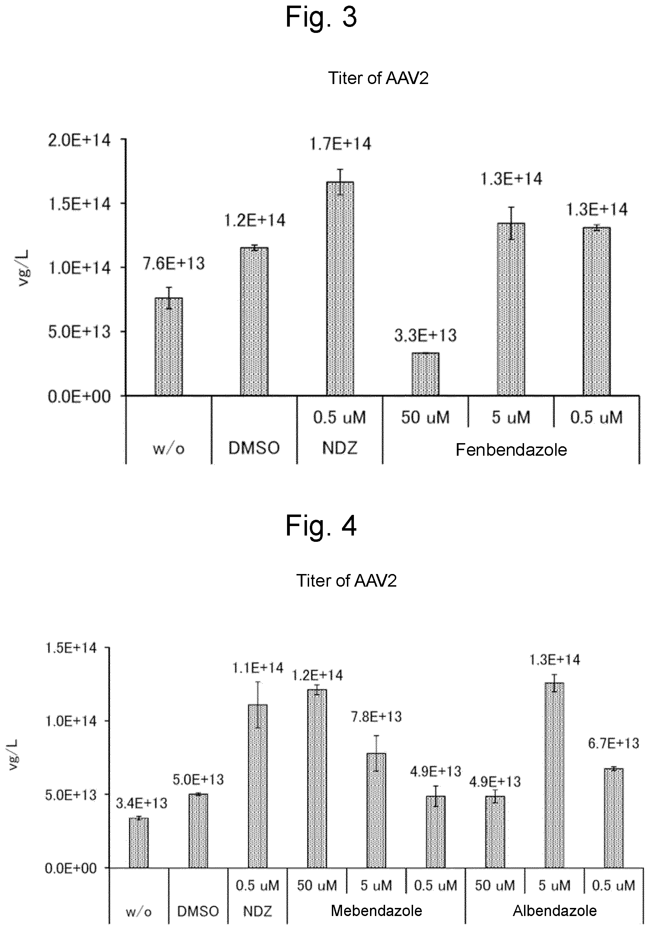

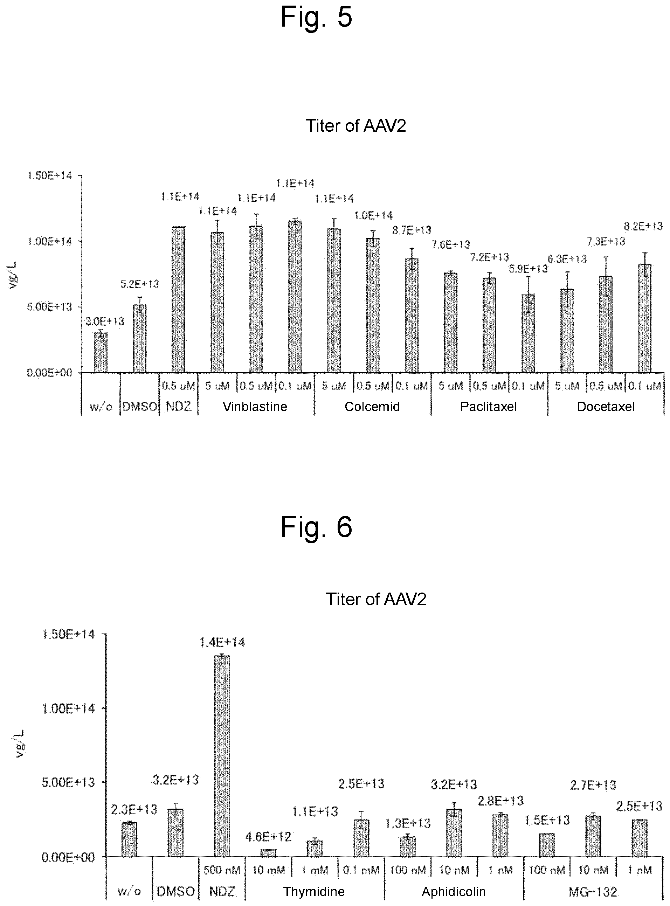

Figure 3 to Figure 7 shows the influence of addition of various cell growth inhibitors A on the titer of the adeno-associated virus vector. Figures 3 to 7 show the titer of the adeno-associated virus vector contained in the cell-derived sample. In Figure 3 to Figure 7, "w/o" shows that cell growth inhibitor A and the solubilizer were not added. "DMSO" shows that only 50 µL of DMSO was added 4 hours after introduction of the nucleic acid into the cells. "NDZ" shows that nocodazole was added as cell growth inhibitor A. "VPA" shows that sodium valproate was added as cell growth inhibitor A.

The results shown in Figure 3 to Figure 7 indicated that the titer of the adeno-associated virus vector is improved by using not only nocodazole, but also, as cell growth inhibitor A, a benzimidazole derivative such as albendazole, mebendazole, or fenbendazole, a vinca alkaloid compound such as vinblastine, a colchicine derivative such as colcemid, or a taxane derivative such as paclitaxel or docetaxel, serving as a compound inhibiting progression of cell cycle in G2 phase or M phase and also as a compound inhibiting microtubule polymerization or a compound stabilizing microtubules.

### <Comparative Example 1: VPA (sodium valproate)>

Nucleic acids were introduced into cells with the same procedure as in Example 1, the adeno-associated virus vector was produced in the condition where sodium valproate (VPA, Tokyo Chemical Industry Co., Ltd.) was added as an agent for inducing viral vector production which does not amount to cell growth inhibitor A, and the titer was measured. The sodium valproate was added to the cell culture medium with water as a solubilizer so that the final concentration of the sodium valproate was 1 mM, 2.5 mM, 5 mM, 10 mM, or 12.5 mM. Nocodazole (Cayman Chemical Company), which amounts to cell growth inhibitor A, was used as control. The nocodazole was added with DMSO as a solubilizer so that the final concentration of nocodazole was 500 nM.

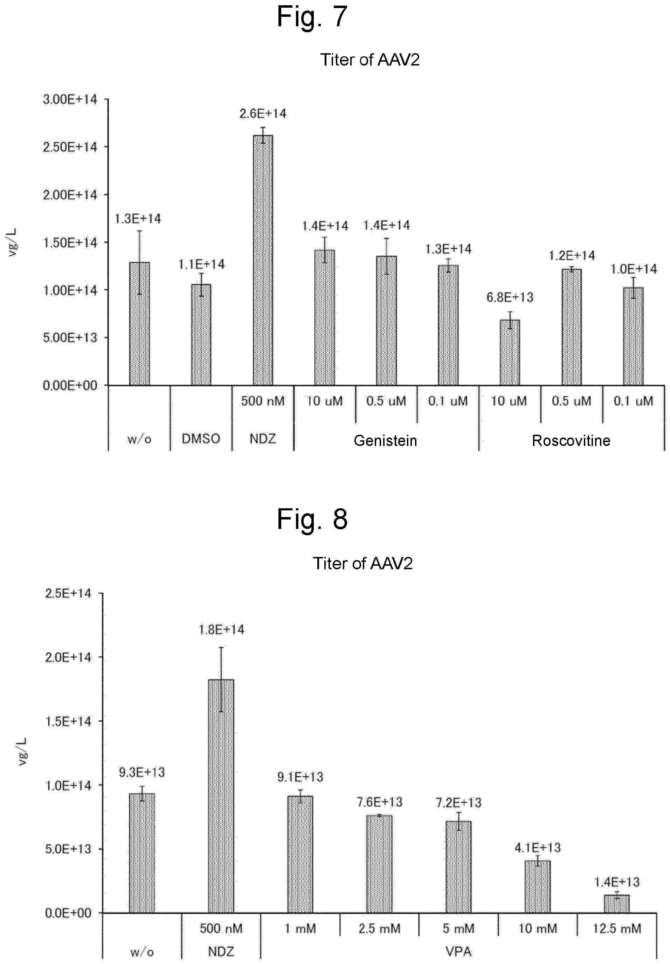

Figure 8 shows the comparison of the influence of addition of cell growth inhibitor A or VPA (sodium valproate) on the titer of the adeno-associated virus vector. Figure 8 shows the titer of the adeno-associated virus vector contained in the cell-derived sample. In Figure 8, "w/o" shows that cell growth inhibitor A and a solubilizer were not added. "NDZ" shows that nocodazole was added as cell growth inhibitor A. "VPA" shows that sodium valproate was added. The results shown in Figure 8 indicated that the effect of improving the titer of the adeno-associated virus vector is larger in an agent for inducing viral vector production comprising cell growth inhibitor A, than an agent for inducing viral vector production comprising sodium valproate (HDAC inhibitor).

### <Example 4: Production of the adeno-associated virus vector with a bioreactor>

This Example 4 corresponds to all of the method of producing a viral vector 1, the method of producing a viral vector 2, the method of inducing viral vector production 1, and the method of inducing viral vector production 2.

On the day of introduction of a nucleic acid into the cells, the suspension HEK293 cells were seeded in a bioreactor (animal cell culture apparatus BCP, volume 1 L, ABLE Corporation), with the number of cells being adjusted to 1 × 10⁶ cells/mL (an amount of culture medium of 400 mL).

pRC2-mi342 (1 µg/µL, 240 µL, packaging plasmid DNA), pAAV-Venus (1 µg/µL, 80 µL, vector plasmid DNA), pHelper (1 µg/µL, 160 µL, helper plasmid DNA), and PEI MAX (1 µg/µL, 960 µL, Polysciences Inc.) as polyethylenimine (PEI) were mixed and left to stand in Opti-MEM (Thermo Fisher Scientific K.K.) for production of a double-stranded circular plasmid DNA-PEI mixed solution.

Nucleic acids were introduced into the cells by adding the double-stranded circular plasmid DNA-PEI mixed solution to the cell culture medium. Thereafter, AAV2 was produced by culturing at 37°C in the presence of 8% CO₂ for 4 days.

Four days after introduction of the nucleic acid into the cells, the supernatant and the cells were separated, and PEG8000 (Sigma-Aldrich Co. LLC) was added to the supernatant obtained, and the adeno-associated virus vector was concentrated. Triton X-100 (Sigma-Aldrich Co. LLC) was added to the residual for cell lysis, and thereafter KANEKA Endonuclease (KANEKA CORPORATION) was added for degradation of DNA. EDTA (NIPPON GENE CO., LTD.) was added and centrifugation was performed, and the adeno-associated virus vector was obtained (supernatant-derived sample).

Triton X-100 (Sigma-Aldrich Co. LLC) was added to the resulting cells for cell lysis, and thereafter KANEKA Endonuclease (KANEKA CORPORATION) was added for degradation of nucleic acids. EDTA (NIPPON GENE CO., LTD.) was added and centrifugation was performed, and the adeno-associated virus vector was obtained (cell-derived sample).

Quantification of the adeno-associated virus was performed by the same method as in Example 1.

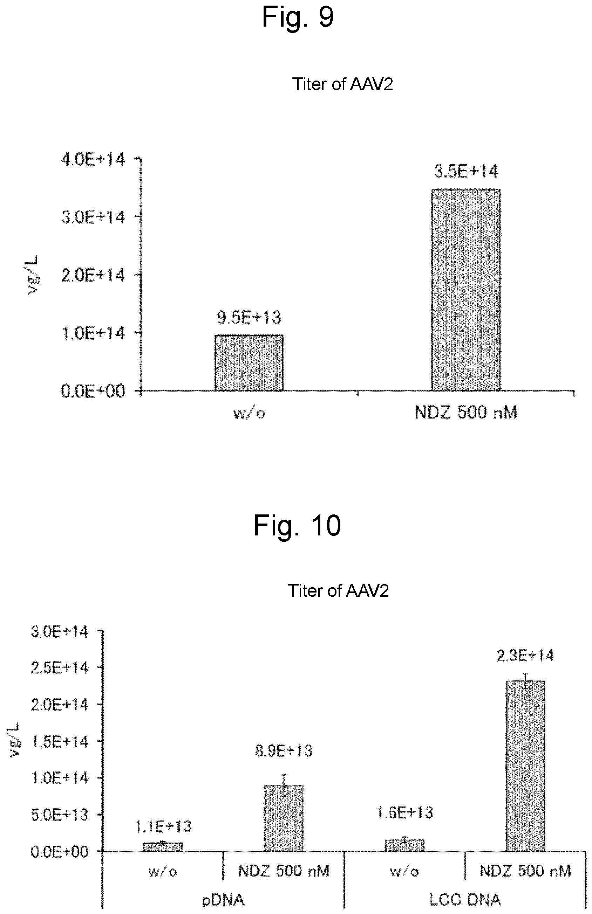

Figure 9 shows the influence of the presence of addition of nocodazole as cell growth inhibitor A on the titer of the adeno-associated virus vector produced using the bioreactor. Figure 9 shows the total titer of adeno-associated virus vector contained in the cell-derived sample and the supernatant-derived sample. In Figure 9, "w/o" shows that cell growth inhibitor A and a solubilizer were not added. "NDZ 500 nM" shows that adjustment to 50 µM was carried out with nocodazole as cell growth inhibitor A and DMSO as a solubilizer, and an amount (4 mL) corresponding to 1% relative to an amount of the cell culture medium of 400 mL was added 4 hours after introduction of the nucleic acid into the cells such that the final concentration of nocodazole was 500 nM.

The results shown in Figure 9 indicated that the results of improvement of the titer of the adeno-associated virus vector by cell growth inhibitor A in Examples 1 to 3 were reproduced also at a bioreactor scale, and the adeno-associated virus vector can be produced and induction of the production thereof can be achieved with cell growth inhibitor A.

### <Example 5: Production of adeno-associated virus vector with cell growth inhibitor A (containing the cell growth inhibitor a) and LCC DNA>

This Example 5 corresponds to all of the method of producing a viral vector 1, the method of producing a viral vector 2, the method of inducing viral vector production 1, and the method of inducing viral vector production 2.

On the day of introduction of the nucleic acid into the cells, the suspension HEK293 cells were seeded in a 50-mL centrifuge tube equipped with a filter cap, with the number of cells being adjusted to 1 × 10⁶ cells/mL (an amount of culture medium of 5 mL).

In the condition where a double-stranded circular plasmid DNA was used as a nucleic acid to be introduced into the cells, pRC2-mi342 (1 µg/µL, 4 µL, packaging plasmid DNA), pAAV-Venus (1 µg/µL, 1 µL, vector plasmid DNA), pHelper (1 µg/µL, 1 µL, helper plasmid DNA), and PEI MAX (1 µg/µL, 12 µL, Polysciences Inc.) as polyethylenimine (PEI) were mixed and left to stand in Opti-MEM (Thermo Fisher Scientific K.K.) for production of a double-stranded circular plasmid DNA-PEI mixed solution. Nucleic acids were introduced into the cells by adding the double-stranded circular plasmid DNA-PEI mixed solution to the cell culture medium. Thereafter, AAV2 was produced by culturing at 37°C in the presence of 8% CO₂ for 4 days.

In the condition where a linear covalently closed DNA was used as the nucleic acid to be introduced into the cells, RC2_LCC_DNA (1 µg/µL, 4 µL, packaging plasmid DNA), Venus LCC DNA (1 µg/µL, 1 µL, vector plasmid DNA), Helper LCC DNA (1 µg/µL, 1 µL, helper plasmid DNA), and PEI MAX (1 µg/µL, 12 µL, Polysciences Inc.) as polyethylenimine (PEI) were mixed and left to stand in Opti-MEM (Thermo Fisher Scientific K.K.) for production of a LCC DNA-PEI mixed solution. Nucleic acids were introduced into the cells by adding the LCC DNA-PEI mixed solution to the cell culture medium. Thereafter, AAV2 was produced by culturing at 37°C in the presence of 8% CO₂ for 4 days.

Extraction and quantification of the adeno-associated virus vector were carried out by the same methods as in Example 1.

Figure 10 shows the titer of the adeno-associated virus vector contained in the cell-derived sample. In Figure 10, "pDNA" shows that a double-stranded circular plasmid DNA was used as the nucleic acid to be introduced into the cells, "LCC DNA" shows that a linear covalently closed DNA was used as the nucleic acid to be introduced into the cells, "w/o" shows that cell growth inhibitor A and a solubilizer were not added, and "NDZ 500 nM" shows that nocodazole as cell growth inhibitor A and DMSO as a solubilizer was added 1 minute after introduction of the nucleic acid into the cells by the same method as in Example 2 so that the final concentration of nocodazole was 500 nM.

The cell growth inhibitor A increased the titer of the adeno-associated virus vector eightfold in the condition where a double-stranded circular plasmid DNA was used, whereas it increased the titer fourteenfold in the condition where a linear covalently closed DNA was used, indicating that the effect of the cell growth inhibitor A was remarkably improved under the condition where the linear covalently closed DNA was used. Thus, it was shown that use of the cell growth inhibitor A and a linear covalently closed DNA in combination is favorable for production of an adeno-associated virus vector.

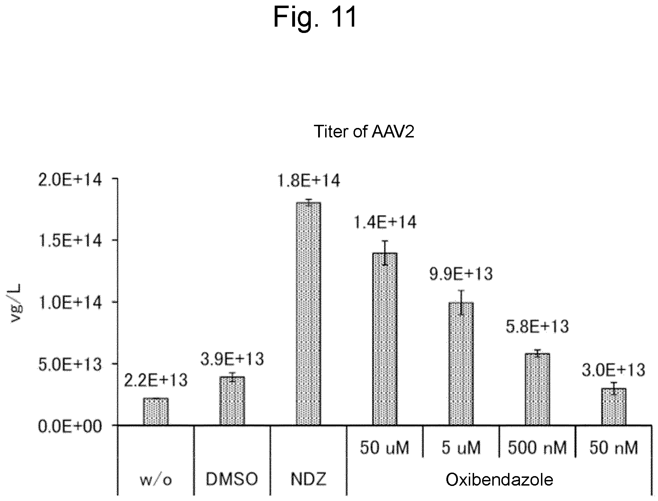

Figure 11 shows the total titer of the adeno-associated virus vector obtained from the cell-derived sample in the condition where the LCC DNA was used as the nucleic acid to be introduced into the cells. In Figure 11, "w/o" shows that cell growth inhibitor A and a solubilizer were not added, "DMSO" shows that only 50 µL of DMSO was added 1 minute after introduction of the nucleic acid into the cells. "NDZ" shows that nocodazole as cell growth inhibitor A and DMSO as a solubilizer were added 1 minute after introduction of the nucleic acid into the cells by the same method as in Example 2 such that the final concentration of nocodazole was 500 nM. When the cell growth inhibitor A was oxibendazole (Tokyo Chemical Industry Co., Ltd.), DMSO was used as a solubilizer and oxibendazole was added to the cell culture medium so that the final concentrations of oxibendazole were four kinds of concentrations, *i.e*., 50 µM, 5 µM, 500 nM, and 50 nM. The titer of the adeno-associated virus vector was increased not only in the condition where nocodazole was used, but also in the condition where oxibendazole was used, indicating that the cell growth inhibitor A which is favorable to be used in combination with a linear covalently closed DNA for the production of an adeno-associated virus is not limited to nocodazole.

### <Example 6: Production of adeno-associated virus vector with cell growth inhibitor A (containing the cell growth inhibitor a) and a solubilizer other than DMSO>

Example 6 corresponds to all of the method of producing a viral vector 1, the method of producing a viral vector 2, the method of inducing viral vector production 1, and the method of inducing viral vector production 2.

On the day of introduction of the nucleic acid into the cell, the suspension HEK293 cells were seeded in a 50-mL centrifuge tube equipped with a filter cap, with the number of cells being adjusted to 1 × 10⁶ cells/mL (an amount of culture medium of 5 mL).

pRC2-mi342 (1 µg/µL, 4 µL, packaging plasmid DNA), pAAV-Venus (1 µg/µL, 1 µL, vector plasmid DNA), pHelper (1 µg/µL, 1 µL, helper plasmid DNA), and PEI MAX (1 µg/µL, 12 µL, Polysciences Inc.) as polyethylenimine (PEI) were mixed and left to stand in Opti-MEM (Thermo Fisher Scientific K.K.) for production of a double-stranded circular plasmid DNA-PEI mixed solution.

Nucleic acids were introduced into the cells by adding the double-stranded circular plasmid DNA-PEI mixed solution to the cell culture medium. Thereafter, AAV2 was produced or the production thereof was induced by culturing at 37°C in the presence of 8% CO₂ for 4 days. Nocodazole (Cayman Chemical Company) as cell growth inhibitor A, and hydrochloric acid, formic acid aqueous solution or ethanol as a solubilizer were added to the cell culture medium 1 minute after introduction of the nucleic acid into the cells so that the final concentration of nocodazole was 500 nM.

Extraction and quantification of the adeno-associated virus vector were carried out by the same methods as in Example 1.

Examples of aspects of the present invention include, *e.g.,* the following.
<1> An agent for inducing viral vector production, comprising cell growth inhibitor a, wherein said cell growth inhibitor a comprises a compound which inhibits progression of cell cycle in G2 phase or M phase.
<2> The agent for inducing viral vector production of <1>, wherein said compound which inhibits progression of cell cycle in G2 phase or M phase comprises a compound which inhibits microtubule polymerization or a compound which stabilizes microtubules.
<3> The agent for inducing viral vector production of <1> or <2>, wherein said compound which inhibits progression of cell cycle in G2 phase or M phase comprises a benzimidazole derivative, a vinca alkaloid compound, or a colchicine derivative.
<4> The agent for inducing viral vector production of any one of <1> to <3>, wherein said compound which inhibits progression of cell cycle in G2 phase or M phase comprises at least any one selected from nocodazole, albendazole, mebendazole, vinblastine, colcemid, thiabendazole, fenbendazole, triclabendazole, flubendazole, oxibendazole, parbendazole, paclitaxel, and docetaxel.
<5> The agent for inducing viral vector production of any one of <1> to <4>, comprising water, hydrochloric acid, formic acid aqueous solution, ethanol, or DMSO as a solubilizer.
<6> The agent for inducing viral vector production of any one of <1> to <5>, wherein said viral vector is an adeno-associated virus vector.
<7> A method of producing a viral vector, wherein said method uses the agent for inducing viral vector production of any one of <1> to <6>.
<8> A method of producing a viral vector, comprising a step of introducing a nucleic acid into a cell, and a step of adding cell growth inhibitor A to said cell, wherein said cell growth inhibitor A is added between 6 hours before and 6 hours after the time of introducing said nucleic acid.
<9> The method of producing a viral vector of <8>, wherein said cell growth inhibitor A, and water, hydrochloric acid, formic acid aqueous solution, ethanol, or DMSO as a solubilizer are added in the step of adding cell growth inhibitor A to said cell.
<10> The method of producing a viral vector of <8> or <9>, wherein said viral vector is an adeno-associated virus vector.
<11> The method of producing a viral vector of any one of <8> to <10>, wherein said nucleic acid is a linear covalently closed DNA.
<12> A method of inducing viral vector production, wherein said method uses the agent for inducing viral vector production of any one of <1> to <6>.
<13> A method of inducing viral vector production, comprising a step of introducing a nucleic acid into a cell, and a step of adding cell growth inhibitor A to said cell, wherein said cell growth inhibitor A is added between 6 hours before and 6 hours after the time of introducing said nucleic acid.
<14> The method of inducing viral vector production of <13>, wherein said cell growth inhibitor A, and water, hydrochloric acid, formic acid aqueous solution, ethanol, or DMSO as a solubilizer are added in the step of adding cell growth inhibitor A to said cell.
<15> The method of inducing viral vector production of <13> or <14>, wherein said viral vector is an adeno-associated virus.
<16> The method of inducing viral vector production of any one of <13> to <15>, wherein said nucleic acid is a linear covalently closed DNA.

The present international application claims the priority based on Japanese Patent Application No. 2021-162665 filed on October 1, 2021, and the entire content of Japanese Patent Application No. 2021-162665 is incorporated into the present international application.

## Claims

1. An agent for inducing viral vector production, comprising cell growth inhibitor a, wherein said cell growth inhibitor a comprises a compound which inhibits progression of cell cycle in G2 phase or M phase.

2. The agent for inducing viral vector production of claim 1, wherein said compound which inhibits progression of cell cycle in G2 phase or M phase comprises a compound which inhibits microtubule polymerization or a compound which stabilizes microtubules.

3. The agent for inducing viral vector production of claim 1 or 2, wherein said compound which inhibits progression of cell cycle in G2 phase or M phase comprises a benzimidazole derivative, a vinca alkaloid compound, or a colchicine derivative.

4. The agent for inducing viral vector production of any one of claims 1 to 3, wherein said compound which inhibits progression of cell cycle in G2 phase or M phase comprises at least any one selected from nocodazole, albendazole, mebendazole, vinblastine, colcemid, thiabendazole, fenbendazole, triclabendazole, flubendazole, oxibendazole, parbendazole, paclitaxel, and docetaxel.

5. The agent for inducing viral vector production of any one of claims 1 to 4, comprising water, hydrochloric acid, formic acid solution, ethanol, or DMSO as a solubilizer.

6. The agent for inducing viral vector production of any one of claims 1 to 5, wherein said viral vector is an adeno-associated virus.

7. A method of producing a viral vector, wherein said method uses the agent for inducing viral vector production of any one of claims 1 to 6.

8. A method of producing a viral vector, comprising
a step of introducing a nucleic acid into a cell, and
a step of adding cell growth inhibitor A to said cell,
wherein said cell growth inhibitor A is added between 6 hours before and 6 hours after the time of introducing said nucleic acid.

9. The method of producing a viral vector of claim 8, wherein:
said cell growth inhibitor A, and
water, hydrochloric acid, formic acid aqueous solution, ethanol, or DMSO as a solubilizer
are added in the step of adding cell growth inhibitor A to said cell.

10. The method of producing a viral vector of claim 8 or 9, wherein said viral vector is an adeno-associated virus vector.

11. The method of producing a viral vector of any one of claims 8 to 10, wherein said nucleic acid is a linear covalently closed DNA.

12. A method of producing a viral vector, wherein said method uses the agent for inducing viral vector production of any one of claims 1 to 6.

13. A method of inducing viral vector production, comprising
a step of introducing a nucleic acid into a cell, and
a step of adding cell growth inhibitor A to said cell,
wherein said cell growth inhibitor A is added between 6 hours before and 6 hours after the time of introducing said nucleic acid.

14. The method of inducing viral vector production of claim 13, wherein:
said cell growth inhibitor A, and
water, hydrochloric acid, formic acid aqueous solution, ethanol, or DMSO as a solubilizer
are added in the step of adding cell growth inhibitor A to said cell.

15. The method of inducing viral vector production of claim 13 or 14, wherein said viral vector is an adeno-associated virus.

16. The method of inducing viral vector production of any one of claims 13 to 15, wherein said nucleic acid is a linear covalently closed DNA.
